# EUROPEAN PATENT SPECIFICATION

(11) **EP 2 357 172 B1**
(45) Date of publication and mention of the grant of the patent: **31.10.2012**
(21) Application number: 11159364.6
(22) Date of filing: 24.12.2007
(51) Int. Cl.: C07D 211/52

(54) **Process for the preparation of pimozide**
Verfahren zur Herstellung von Pimozid
Procédé pour la préparation de pimozide

(30) Priority: 28.12.2006 ES 200700030
(43) Date of publication of application: 17.08.2011
(62) Divisional of application: 07866264.0
(73) Proprietor: Institut Univ. de Ciència i Tecnologia, S.A., 08100 Mollet del Vallès Barcelona (ES)
(72) Inventor: Estévez Company, Carlos, 08100, MOLLET DEL VALLÈS (ES); Bayarri Ferrer, Navidad, 08100, MOLLET DEL VALLÈS (ES); Castells Boliart, Josep, 08100, MOLLET DEL VALLÈS (ES); Echeverria Beistegui, Begoña, 08100, MOLLET DEL VALLÈS (ES)
(74) Representative: Ponti Sales, Adelaida

(56) References cited:
- DD-A1- 243 284
- JP-A- 50 112 373
- US-A- 3 196 157

## Description

The present invention relates to the field of inert solvents useful for carrying out chemical reactions and, more particularly, to their use for the preparation of pimozide.

### BACKGROUND OF THE INVENTION

An appropriate selection of the solvent in the different reaction or purification steps may enhance the yield of the active ingredient, or determine characteristics such as crystal form, purity, and solubility. Therefore, the solvent is a critical parameter in the preparation of drug substances. Moreover, the level of residual solvents in the end product must be reduced to an acceptable amount in order to meet product specifications according to safety requirements. Guideline for Residual Solvents (CPMP/ICH/283/95) and subsequent revisions establish a classification and the limits of residual solvents based on safety purposes.

Known hazardous solvents should be avoided in the production of both the pharmaceutical active ingredient and the final pharmaceutical product because they may cause unacceptable toxicities or deleterious environmental effects. Examples of these solvents are carbon tetrachloride, 1,2-dichloroethane, 1,1-dichloroethane and 1,1,1-trichloroethane.

Other solvents associated with a lower toxicity level should be limited in order to protect patients from potential adverse effects. Examples of these solvents are xylene, toluene, methanol, hexane, N,N-dimethylformamide (DMF), N,N-dimethylacetamide (DMA), dichloromethane, chloroform and acetonitrile.

Solvents that may be regarded as less toxic are acetone, ethanol, isopropyl alcohol (IPA), ethyl acetate, dimethylsulfoxide (DMSO), ethyl ether, methylethylketone (MEK), methylisobutylketone (MIBK), propanol and tetrahydrofuran (THF). These solvents should be employed when their use can be justified by the process or product characteristics.

Although, due to their lower toxicity, the solvents from the aforesaid third class are the preferred ones, they should be still used according to valid regulations that establish parameters so as to minimize their impact on both human health and environment. Thus, if MIBK is taken as an example, since it is one of the most widely used solvent in the chemical industry due, among other reasons, to its ability to solubilize organic compounds and to the fact that it can be easily removed by evaporation, there may be some risks associated with its use. The intrinsic hazard of MIBK is essentially evidenced by its physical properties (bp: 117-118 °C; vP: 15 mmHg (20°C)) and toxicological data (LD₅₀ (oral-rats): 2.1 g/kg), in such a way that European legislation has established indicative environmental and biological limits. Consequently, Spanish legislation has included MIBK in the general list of environmental limit values of professional exposure and established a daily exposure value (ELV-DE) of 83 mg/m³ and a short exposure value (ELV-SE) of 208 mg/m³. MIBK exhibits the following R-phrases ("Risk Phrases"): 11-20-36/37-66 ("Highly flammable. Harmful by inhalation. Irritating to eyes and to respiratory system. Repeated exposure may cause skin dryness or cracking"). From the viewpoint of safety, MIBK exhibits the following S-phrases: 9-16-29 ("Keep container in a well-ventilated place. Keep away from sources of ignition - No smoking. Do not empty into drains"). The hazard codes of the National Fire Protection Association (NFPA) assign the score of 3 the flammability of MIBK and the score of 2 its hazard to human health.

The primary objective of the development of a pharmaceutical active ingredient is to find one or various synthetic routes which allow to obtain the concerned compounds with an acceptable yield. This implies that suitable reaction conditions, including solvents, should be used in order to achieve such objective at laboratory scale. Moreover, other additional aspects (e.g., solvents to be limited so as to meet drug product, safety, and environmental requirements) should be taken into account in a subsequent stage of process optimization and, specially, in its industrial scale-up.

Thus, in general, it is of a great interest to the pharmaceutical industry the identification and use of alternative solvents in order to minimize the problems associated with solvents that are usually employed in known preparation processes of active ingredients.

Document DD243284 A1 relates to a process for the preparation of pimozide with a yield between 75 and 80% using as reagents 1-chloro-4,4-bis-(4-fluorophenyl)-butane and 1-(4-piperidyl)-2-benzimidazolinone and a mixture of C6-C9 alcohols as a solvent.

Document JP5011373A1 relates to a 2-step process for the preparation of pimozide using as reagents 1-halo-4,4-di-(4-fluorophenyl)butane (4) and 1-(1-benzylpiperidin-4-yl)-1H-benzo[d]imidazol-2(3H)-one and DMF as a solvent.

### DESCRIPTION OF THE INVENTION

Surprisingly, it has been found that glycerol formal allows to carry out reactions for obtaining active pharmaceutical ingredients under highly safe and non-contaminant conditions. Thus, the end product, in addition to fully satisfy the safety requirements established by current regulations due to its low toxicity, shows several additional advantages.

Glycerol formal is a mixture of two isomers, 4-hydroxymethyl-1,3-dioxolane and 5-hydroxy-1,3-dioxane. These two isomers are present in constant proportion of 40% and 60%, respectively. Glycerol formal is very stable under both neutral and basic conditions and is inert toward the solubilized products which are compatible with the alcohol and acetal functions. In a strong acid medium, glycerol formal may be decomposed into formaldehyde and glycerol. In order to avoid its decomposition, the medium pH should preferably be higher than 4. Glycerol formal is completely miscible in ether, acetone, essential oils, alcohols and water. In addition, it is practically odourless, moderately biodegradable and presents a very low toxicity. It is known the use of glycerol formal in the state of the art, such as in the field of casting industry and in manufacturing household products, printing inks and paints. In the pharmaceutical and veterinary field, glycerol formal is employed as an excipient or vehicle in the formulation of products such as injectable solutions. However, nothing related to the use of glycerol formal as an inert solvent in chemical reactions for the preparation or purification of drug products is mentioned or suggested in the state of the art.

Table 1 shows the physicochemical properties of MIBK as compared with those of glycerol formal. The data for MIBK were obtained from the International Chemical Safety Card published by "Instituto Nacional de Seguridad e Higiene en el Trabajo" (National Institute for Occupational Safety and Hygiene). The data for glycerol formal were obtained from the most recent manufacturer's standard safety data sheet.

It can be seen that the boiling point and flash point of glycerol formal are approximately 80°C higher than those of MIBK and that the vapour pressure of glycerol formal is lower than that of MIBK, all of which promotes a lower exposure risk. Moreover, glycerol formal presents a better toxicological profile as evidenced by the absence of R-phrases and environmental limit values, as well as a five times higher lethal dose in rats (ingestion). The less-flammability of glycerol formal, as defined by the hazard codes of the NFPA, offers some advantages to the safety of its manipulation in a chemical plant.

**Table 1. Physicochemical properties of MIBK and Glycerol Formal**

| **PROPERTY** | **MIBK** | **GLYCEROL FORMAL** |
|---|---|---|
| **Colour** | Colourless liquid | Colourless liquid |
| **Odour** | Characteristic odour | Odourless liquid |
| **Boiling point (°C)** | 117-118 | 192-193 |
| **Flash point (°C)** | 18 | 98 |
| **Self-flash point (°C)** | 449 | >400 |
| **Explosivity limits (% v/v)** | 1.7 - 7.6 | Unavailable |
| **Vapour pressure (mmHg)** | 15 (exp., 20°C) | 0.13 (calc., 25°C) |
| **Density (25°C, g/mL)):** | 0.801 | 1.203 |
| **Solubility in H₂O (g/L)** | 20 | Miscible in all proportions |
| **LD₅₀(oral-rats, g/kg)** | 2.1 | 10 |
| **ELV-DE** | 83 mg/m³ 20 ppm | Unavailable |
| **ELV-SE** | 208 mg/m³ 50 ppm | Unavailable |
| **R-Phrases** | 11-20-36/37-66 | None |
| **S-Phrases** | 9-16-29 | None |
| **NFPA Codes** | Health: 2 | Health: 1 |
| | Flammability: 3 | Flammability: 1 |
| | Reactivity: 0 | Reactivity: 0 |

Therefore, an aspect of the present invention concerns a process for the preparation of an active pharmaceutical ingredient, wherein the reaction for obtaining said active ingredient is carried out in glycerol formal as an inert solvent, at a reaction pH higher than 4. It is understood as "inert solvent" that glycerol formal acts only as a solvent without taking part in the reaction as a reagent, without being chemically incompatible with any component of the reaction mixture and without being decomposed under the reaction conditions.

It should be emphasized among the advantages of the use of glycerol formal that it enables to carry out the preparation of active pharmaceutical ingredients under safer, non-contaminant conditions and with high yields. Thus, due to the fact that glycerol formal has a high boiling point, reactions can be carried out at high temperatures, what allows to accelerate or even optimize such reactions which, at lower temperatures, almost do not progress. Notwithstanding that, the use of glycerol formal was found to allow reactions to be carried out comparatively faster than in other solvents even at lower temperatures. Likewise, its high flammability point enables to operate safely at industrial levels and to carry out operations such as filtration at a temperature higher than room temperature or to handle the solvent or the wet products from the solvent with no flammability hazard.

In a preferred embodiment, the preparation of the active pharmaceutical ingredient is performed by a nucleophilic substitution reaction. Preferably, the nucleophilic substitution reaction is of S_{N}2 type.

In a preferred embodiment, the pharmaceutical active ingredient is pimozide, comprising the process for its preparation the reaction 4-(2-oxo-1-benzimidazolinyl)-piperidine (3) and 1-chloro-4,4-di-(4-fluorophenyl)butane (4).

Scheme 2 depicts the process for the preparation of pimozide according to the present invention.

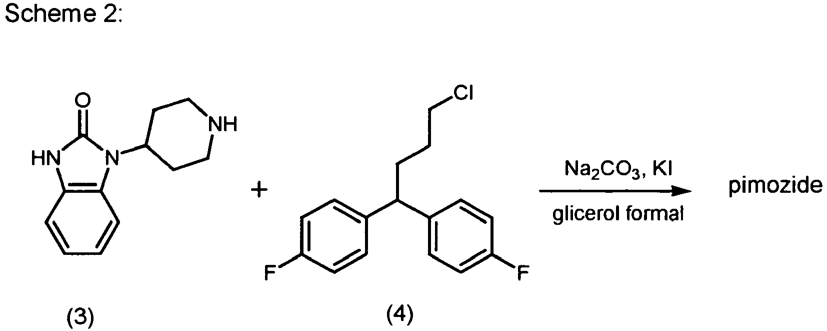

The reaction is preferably carried out in the presence of a base in an amount from 1 to 1.2 equivalents in relation to 4-(2-oxo-1-benzimidazolinyl)-piperidine in order to neutralize the hydrochloric acid released in the course of the reaction. Examples of suitable bases are trialkylamines, heterocyclic tertiary amines and bases from alkaline or alkaline earth metals, such as hydroxides and alkaline or alkaline earthy metal carbonates, such as sodium hydroxide, potassium hydroxide, sodium carbonate and potassium carbonate. Preferably, the base is an alkaline or alkaline earth metal carbonate, even more preferably sodium carbonate. It is similarly convenient to add a catalytic amount of potassium iodide.

In a preferred embodiment, the reaction for obtaining pimozide is carried out at a temperature comprised between 60 and between 85 and 95°C. In an even more preferred embodiment, glycerol formal is present in an amount comprised between 3 and 10 ml per each gram of 4-(2-oxo-1-benzimidazolinyl)-piperidine. More preferably the amount of glycerol formal is comprised between 3 and 5 ml in order to enhance productivity.

Gycerol formal enables the reaction to be carried out comparatively in a considerably faster way than in other solvents even a lower temperature, since the reaction may be completed in less than 10 hours.

Another advantage of the use of glycerol formal in the process for the preparation of pimozide according to the present invention is that isolation of the end product is performed by simple dissolution of salts and glycerol formal in water and subsequent filtration. For comparative purposes, the purification after preparing the active ingredient as described in the patent US 3,196,157 is performed by grinding the solid residue with diisopropyl ether and then the resulting material is recrystallized firstly from acetone/MIBK and finally from acetone.

Table 3 shows the values of different reaction parameters for the preparation of pimozide according to the classical process in comparison with those obtained according to the process of the present invention.

**Table 3**

| | **Classical synthesis** | **Alternative synthesis** |
|---|---|---|
| **Solvent** | MIBK | Glycerol formal |
| **Reagents** | | |
| **(3)** | 1 eq | 1.10 eq |
| **(4)** | 1 eq | 1 eq |
| **Na₂CO₃** | 1.60 eq | 1.10 eq |
| **Time** | 65h | 7h |
| **Temperature** | 120°C | 90°C |
| **Purification** | Diisopropyl ether Acetone/MIBK | H₂O |

The preparation of pimozide by the process of the present invention is much more efficient than the classical process as described in US 3,196,157 because it enables to obtain a 98% yield and exhibits the advantages of requiring fewer base equivalents, a shorter reaction time and a lower reaction temperature. Furthermore, the use of glycerol formal in the process of the present invention allows simplifying the isolation and purification of the product.

Throughout the description and claims the word "comprise" and variations thereof are not intended to exclude other technical features, additives, components, or steps. Additional objects, advantages and features of the invention will become apparent to those skilled in the art upon examination of the description or may be learned by practice of the invention. The following examples and drawings are provided by way of illustration, and is not intended to be limiting of the present invention.

### EXAMPLE

### Example: Preparation of 1-{1-[4,4-bis-(4-fluorophenyl)-butyl]-piperidin-4-yl}-imidazolinyl-2-one (pimozide)

0.963 g (4.3·10⁻³ mole, 1 eq) of 4-(2-oxo-1-benzimidazolinyl)-piperidine0.504 g (4.7·10-³ mole, 1.09 eq) of sodium carbonate, 0.0245 g (0.8% by weight) of potassium iodide and 3 mL of glycerol formal were weighed. 1.489 g (4.8·10⁻³ mols, 1.10 eq) of 1-chloro-4,4-di-(4-fluorophenyl)butane were added. The reaction mixture was stirred at 80 °C. Analysis by thin layer chromatography showed that the reaction had been completed within 7 hours. 3 mL of water were added, then stirred and filtered under vacuum. The resulting solid was washed twice with 2 mL of water. The obtained solid was dried at 40°C under reduced pressure. The title compound was obtained as a white solid. Yield: 97.5%, purity: 99.5%. Rf (MeOH/Acetone 30/70) = 0.67. ¹H NMR (300 MHz, CDCl₃) δ 10.1 (1 H, s, NH), 6.9-7.3 (12 H, m, Ar), 4.1-4.2 (1 H, m, CH), 3.9 (1 H, t, CH, J=7.8 MHz), 3 (2H, d, CH₂, J=11.4 MHz), 2.47 (2H,t, CH₂, J=6MHz), 2.45 (2H, t, CH₂, J=6MHz), 2.07 (2H, q, J=6 MHz), 1.7-1.9 (2H, m),1.47 (2H, m).

## Claims

1. Process for the preparation of pimozide, **characterized in that** the reaction for obtaining said active ingredient is carried out in glycerol formal as an inert solvent at a pH higher than 4 and wherein said reaction is a S_{N}2 nucleophilic substitution.

2. Process according to claim 1, comprising the reaction of 4-(2-oxo-1-benzimidazolinyl)-piperidine and 1-chloro-4,4-di-(4-fluorophenyl)butane.

3. Process according to claim 2, wherein the reaction is carried out in the presence of a base in an amount comprised between 1 and 1.2 equivalents in relation to 4-(2-oxo-1-benzimidazolinyl)-piperidine.

4. Process according to any of claims 2 or 3, wherein potassium iodide is added to the reaction as a catalyst.

5. Process according to any one of claims 2 to 4, wherein the reaction is carried out at a temperature comprised between 60 and 95°C.

6. Process according to claim 5, wherein the reaction is carried out at a temperature comprised between 80 and 95°C.

7. Process according to any one of claims 2 to 6, wherein glycerol formal is present in an amount comprised between 3 and 10 ml per each gram of 4-(2-oxo-1-benzirnidazolinyl)-piperidine.

8. Process according to claim 7, wherein said glycerol formal is present in an amount comprised between 3 and 5 ml per each gram of 4-(2-oxo-1-benzimidazolinyl)-piperidine.

## Patentansprüche

1. Verfahren zur Herstellung von Pimozid, **dadurch gekennzeichnet, dass** die Umsetzung, um den Wirkstoff zu erhalten, in Glycerol Formal als inertes Lösungsmittel bei einem pH-Wert von über 4 durchgeführt wird, und wobei die Umsetzung eine S_{N2} nucleophile Substitution ist.

2. Verfahren nach Anspruch 1, umfassend die Umsetzung von 4-(2-Oxo-1-benzimidazolinyl)-piperidin und 1-Chlor-4,4-di-(4-fluorophenyl) butan.

3. Verfahren nach Anspruch 2, wobei die Umsetzung in Gegenwart einer Base in einer Menge durchgeführt wird, die 1 bis 1,2 Äquivalente in Bezug auf 4-(2-Oxo-1-benzimidazolinyl)-piperidin umfasst.

4. Verfahren nach einem der Ansprüche 2 oder 3, wobei Kaliumiodid zur Reaktion als Katalysator zugegeben wird.

5. Verfahren nach einem der Ansprüche 2 bis 4, wobei die Umsetzung bei einer Temperatur von 60 - 95°C durchgeführt wird.

6. Verfahren nach Anspruch 5, wobei die Umsetzung einer bei Temperatur von 80 - 95°C durchgeführt wird.

7. Verfahren nach einem der Ansprüche 2 bis 6, wobei Glycerol Formal in einer Menge vorliegt, die 3 -10 ml pro jedes Gramm 4-(2-Oxo-1-benzimidazolinyl)-piperidin umfasst.

8. Verfahren nach Anspruch 7, wobei Glycerol Formal in einer Menge vorliegt, die 3 - 5 ml pro jedes Gramm 4-(2-Oxo-1-benzimidazolinyl)-piperidin umfasst.

## Revendications

1. Procédé de préparation de pimozide, **caractérisé en ce que** la réaction pour l'obtention dudit ingrédient actif est réalisée dans du glycérol formal en tant que solvant inerte à un pH supérieur à 4, ladite réaction étant une substitution nucléophile S_{N}2.

2. Procédé selon la revendication 1, comprenant la réaction de 4-(2-oxo-1-benzimidazolinyl)-pipéridine et de 1-chloro-4,4-di-(4-fluorophényl)butane.

3. Procédé selon la revendication 2, dans lequel la réaction est réalisée en présence d'une base en une quantité comprise entre 1 et 1,2 équivalent par rapport à la 4-(2-oxo-1-benzimidazolinyl)-pipéridine.

4. Procédé selon l'une quelconque des revendications 2 ou 3, dans lequel de l'iodure de potassium est ajouté à la réaction en tant que catalyseur.

5. Procédé selon l'une quelconque des revendications 2 à 4, dans lequel la réaction est réalisée à une température comprise entre 60 et 95 °C.

6. Procédé selon la revendication 5, dans lequel la réaction est réalisée à une température comprise entre 80 et 95 °C.

7. Procédé selon l'une quelconque des revendications 2 à 6, dans lequel le glycérol formal est présent en une quantité comprise entre 3 et 10 ml pour chaque gramme de 4-(2-oxo-1-benzimidazolinyl)-pipéridine.

8. Procédé selon la revendication 7, dans lequel ledit glycérol formal est présent en une quantité comprise entre 3 et 5 ml pour chaque gramme de 4-(2-oxo-1-benzimidazolinyl)-pipéridine.
